Europäisches Patentamt

European Patent Office

Office européen des brevets

(11) Publication number: **0 573 599 B1**

# EUROPEAN PATENT SPECIFICATION

(45) Date of publication of patent specification: **24.05.95**

(51) Int. Cl.[6]: **C30B 30/08**, C30B 29/58, C30B 7/00, C07K 1/30

(21) Application number: **92908432.5**

(22) Date of filing: **24.02.92**

(86) International application number: **PCT/US92/01125**

(87) International publication number: **WO 92/14869 (03.09.92 92/23)**

(54) **CRYSTALLIZING BIOLOGICAL MACROMOLECULES IN MICROGRAVITY.**

(30) Priority: **25.02.91 US 659948**
**18.02.92 US 836658**

(43) Date of publication of application:
**15.12.93 Bulletin 93/50**

(45) Publication of the grant of the patent:
**24.05.95 Bulletin 95/21**

(84) Designated Contracting States:
**AT BE CH DE DK ES FR GB GR IT LI LU MC NL SE**

(56) References cited:
**EP-A- 0 303 224**
**EP-A- 0 332 491**

**Journal of Crystal Growth, vol. 76, 1986, Amsterdam, NL; W. LITTKE et al.: "Protein single crystal growth under microgravity", pp. 663-672**

(73) Proprietor: **SCHERING CORPORATION**
**2000 Galloping Hill Road**
**Kenilworth**
**New Jersey 07033 (US)**

Proprietor: **THE UAB RESEARCH FOUNDATION**
**113 Mortimer Jordan Hall,**
**1825 University Blvd.**
**Birmingham, AL 35209-2010 (US)**

(72) Inventor: **BUGG, Charles, E.**
**4370 Cliff Road**
**Birmingham, AL 35200 (US)**
Inventor: **DE LUCAS, Lawrence, J.**
**5533 Afton Drive**
**Birmingham, AL 35200 (US)**
Inventor: **NAGABHUSHAN, Tattanahalli, L.**
**3 Sunset Lane**
**Parsippany, NJ 07054 (US)**
Inventor: **TROTTA, Paul, P.**
**2429 Harmod Cove Towers**
**Secaucus, NJ 07094 (US)**

(74) Representative: **Ritter, Stephen David et al**
**Mathys & Squire**
**100 Grays Inn Road**
**London WC1X 8AL (GB)**

EP 0 573 599 B1

**Description**

BACKGROUND OF THE INVENTION

One need for purified protein is derived from the pharmaceutical industry's interest in protein products as therapeutic agents. Protein therapeutic agents can be used in humans against any number of diseases, including cancer, immunodeficiency diseases and various types of infections. However, since many medicinal proteins are prepared from genetically engineered bacteria, procaryotic protein impurities can present costly problems in final purification steps. It is essential to remove these contaminants because they are immunogenic. If the contaminants are allergenic, even minute amounts can cause significant adverse side reactions. Accordingly, protein therapeutic agents must be extremely pure.

Over the past ten years there has been an exponential growth in protein pharmaceuticals. Human insulin, interferons, human growth hormone, TPA (tissue plasminogen activator) and erythropoietin are examples. This is so mainly because of genetic engineering techniques which allow for the large scale microorganism synthesis of proteins that occur in minute amounts in the human body.

It is known to grow protein crystals in microgravity using hanging drops in a vapor diffusion apparatus (VDA). However, the disadvantage of this method is that it produces limited amounts of crystals because the volume of the hanging drop is small, i.e., 0.05 ml. The yield is sufficient to supply research amounts of the protein crystals for x-ray crystallography, but cannot be considered a large scale method that could provide bulk amounts of protein crystals. Another limitation of the hanging drop method, which again is due to the small sample size, is the problem of droplet surface effects. Flow patterns on the surface may adversely affect protein crystal growth.

From EP-A-0 303 224 an apparatus and a method for crystallizing proteins is known.

**SUMMARY OF THE INVENTION**

According to one aspect of the present invention there is provided an apparatus capable of producing macromolecular crystals in microgravity comprising:

(a) at least one container having opposing ends and a thermally conductive lid at one of said opposing ends;

(b) at least one bottle disposed inside the container and having a thermally conductive cap such that the cap contacts the lid so as to allow heat transfer therebetween; and

(c) means for maintaining the lid in contact with a variable heat source such that a temperature gradient is obtainable inside said bottle.

The invention further provide a method for producing commercial scale quantities of macromolecular crystals of at least 0.5 mg comprising:

(a) providing at least one bottle having a thermally conductive cap;

(b) loading a pre-mixed aqueous solution of a liquid biological macromolecule and a precipitating agent into the bottle and fixing said cap onto said bottle after loading the solution into said bottle;

(c) placing said thermally conductive cap in contact with a variable heat source such that the cap contacts the variable heat source so as to allow heat transfer therebetween; and

(d) changing the temperature of said variable heat source while said bottle is in the microgravity environment so as to establish a temperature gradient in the bottle sufficient to crystallize the biological macromolecule wherein the microgravity grown crystals thereby produced are characterized by at least one of the group consisting of higher Bragg angles for the diffraction limit less thermal vibration, and less atomic and molecular lattice misorientation as reflected by the positive $\Delta B$ slope displayed on a relative Wilson plot when compared to earth-grown crystals of the same biological macromolecule.

The present invention permits large-scale production of biological macromolecular crystals, which can be useful as therapeutic agents, in the microgravity environment of space. Accordingly, the present invention provides an apparatus capable of producing macromolecular crystals in microgravity, comprising: at least one bottle having a temperature-conducting cap; at least one container having a temperature-conducting lid, wherein the bottle is disposed inside the container such that the cap engages the lid so as to allow heat transfer therebetween; and means for maintaining the lid in contact with a variable heat source such that a temperature gradient is obtainable inside the bottle.

The present invention also provides a method for producing macromolecular crystals comprising changing the temperature in a microgravity environment at one end of a pre-mixed aqueous solution of a liquid biological macromolecule and a precipitating agent to effect a temperature gradient therethrough sufficient to crystallize the biological macromolecule. By growing crystals in a static (non-agitated) solution under microgravity conditions ($10^{-2}$ to $10^{-6}$ g), disadvantageous convection currents in the solution itself are avoided, permitting temperature changes to effect linear temperature gradients in the solution. Accordingly, crystallization in accordance with the present invention effects large-scale quantities of larger, more highly ordered, and more pure crystals than can be grown on earth.

BRIEF DESCRIPTION OF THE DRAWINGS

The following figures are provided to illustrate a preferred embodiment of the present invention.

Fig. 1 is a partially-exploded perspective view according to the preferred embodiment of the invention;

Fig. 1A is a perspective view showing the torquing tool;

Fig. 1B is a perspective view of showing the t-handle;

Fig. 2 is a perspective view of the aluminum assembly for the PCF with all of the cylinders in place according to the preferred embodiment;

Fig. 3 is a cross-sectional taken along line 3-3 in Figure 1;

Fig. 4 is perspective view showing insertion of the aluminum assembly in the refrigerator-incubator module (RIM);

Fig. 5 is a perspective view showing an exemplary PCF with cap torqued to 130 lbs/in and an aluminum cylinder top;

Fig. 6 is an exploded perspective view showing an aluminum cylinders and its stem-and-purge valve and the stem-and-purge cap;

Fig. 7 is a perspective view showing the assembly with the front panel in place from the left side;

Fig. 8 is a side elevational view showing the assembly with the front panel in place from the right side;

Fig. 9 is a bottom plan view of the assembly;

Fig. 10 is a ton plan view of the RIM showing the pressure release valve;

Figure 11 is a graphical representation of the length and width of free-floating "rosette" crystals in flight and on ground in one experiment using the present invention;

Figure 12 is a graphical representation of "rosette" crystal size in flight and on ground on the side of the PCF relative to its location in the experiment of Figure 11;

Figure 13 is a graphical representation of the length and width of free-floating single crystals in flight and on ground in the experiment of Figure 11;

Figure 14 is a graphical representation of single crystal size in flight and on ground on the side of the PCF relative to its location in the experiment of Figure 11;

Figure 15 is a graphical representation of the length and width of free-floating single crystals in flight and on ground in another experiment using the present invention; and

Figure 16 is a graphical representation of single crystal size in flight and on ground on the side of the PCF relative to its location in the experiment of Figure 15.

DETAILED DESCRIPTION OF THE PREFERRED EMBODIMENTS

In a preferred embodiment of the present invention, the apparatus comprises a set of four polysulfone bottles of identical diameter and varying heights corresponding to internal volumes of 500, 200, 100 and 50 milliliters (ml). These four bottles each have a metal cap that apposes a thermal unit to drive the temperature change. Because each unit is separate and insulated from the other, each has a different temperature gradient.

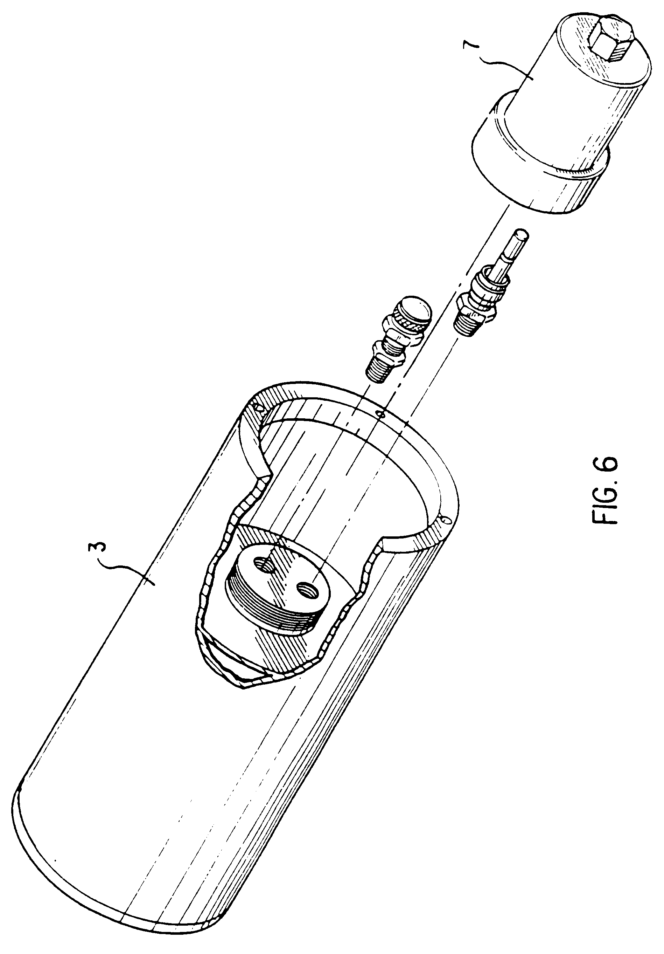

The device of the invention is made up of four polysulfone bottles (PCFs) 1 with aluminum PCF caps 2 capable of containing a protein solution to be crystallized, four aluminum cylinders 3 having aluminum cylinder tops 3a at one end and stem and purge valves at the other end, two side plates 4a-b, one front panel 4c, attachment hardware including rails 6 and springs, and four stem-and-purge caps 7 (see fig. 6).

The apparatus comprises four PCFs 1 which are heated or cooled at one end to initiate protein crystal growth. The PCF bottles are made of polysulfone, which has the advantages of being qualified for space flight, inert to protein adhesion, transparent and pliant enough to withstand temperature fluctuations in the 4°C to 60°C range. Although PS-1700 polysulfone is preferably used, any material which is non- adherent to proteins could be used for the PCF bottles. The four PCF bottles 1 are of the same diameter but of different heights and, accordingly, different volumes. The different heights allow a different temperature gradient to be obtained in each bottle. Once the appropriate temperature gradient has been determined for a particular protein, bottles of same height and different diameters can be used.

The volumes of the preferred embodiment are 500, 200, 100 and 50 ml, but the bottles can be of any volume as long as they fit inside of the RIM 5 or other thermally controllable unit. Each is closed with a threaded metal V-shaped cap 2 using a single O-ring so that the cap 2 serves as the pathway for heating or cooling. The "V-shape" of the bottom portion of the cap 2 functions to push bubbles out of the bottle when the cap is being placed thereon. The RIM itself is a known device. It is a solid-state (semiconductor) heating and cooling device designed to regulate an internal cavity temperature adjustable to within $0.1 °C$ with an external knob adjustment.

Specifically, the RIM is a six-sided box, one side of which is a door. The six sides comprise a double-walled structure, the external walls of which are reinforced fiberglass and the interior walls of which are heat-conducting metal. Five of the six double walls contain insulation therein. The sixth double wall, the interior wall of which is termed the cold plate, houses a thermal control unit (TCU). The TCU is a well known device made up of a thermal electric unit (TEU), a heat exchanger, a temperature-feed-back sensor, and a fan.

The TEU is a well known plate-like unit sandwiched between the cold plate and the heat exchanger, also plate-like. Depending upon the direction in which electric current passes through the TCU, heat is either applied to or withdrawn from the cold plate, thereby regulating the temperature in the internal chamber of the RIM. The TCU operates to maintain the temperature inside the chamber within $0.5 °C$ of the set temperature, which is set between $0-40 °C$ by a knob on the outside of the RIM equipped with a digital temperature readout. In a normal horizontal position, the RIM door, the interior wall of which carries a rubber gasket, is hinged on the bottom, and the cold plate is the left vertical wall of the internal chamber. For structural support, the RIM is housed inside a metal frame covering the edges of the RIM box and joined at the corners.

The foregoing embodiment of the present invention using bottles of different size allows evaluation of crystal size and quality as a function of the different temperature gradients obtained because different proteins require different gradients for optimal crystal growth.

Aluminum cylinders 3 are used to hold the PCFs in place and apposed to a thermal control device of the RIM 5. The cylinders 3 are provided with aluminum cylinder tops 3a to which the PCFs are attached using four screws 8. In this way, the bottles are cantilevered within the cylinders, thus allowing ample air insulation around the bottle within each cylinder. The stem and purge valves are used for ground-based leak testing only and are securely covered by stem-and-purge caps 7 when the apparatus is in use.

Each cylinder 3 is a cylindrical, hermetically sealed vessel sandwiched between aluminum side plates 4a-b and fastened to them with screws 10. An aluminum front 4c is attached at one end of the side plates 4a-b. Each cylinder is identical in size. The primary functions of the cylinders are to thermally isolate the PCFs from each other, to retain the bottle contents and the bottle in the event that the bottle leaks or fails structurally, and to hold the PCF in the RIM 5 for thermal control. For the latter function, the cylinders pass through the left side plate 4a so that the aluminum cylinder top 3a, and thus the aluminum PCF cap 2, are in direct thermal contact with the RIM 5 thermal plate.

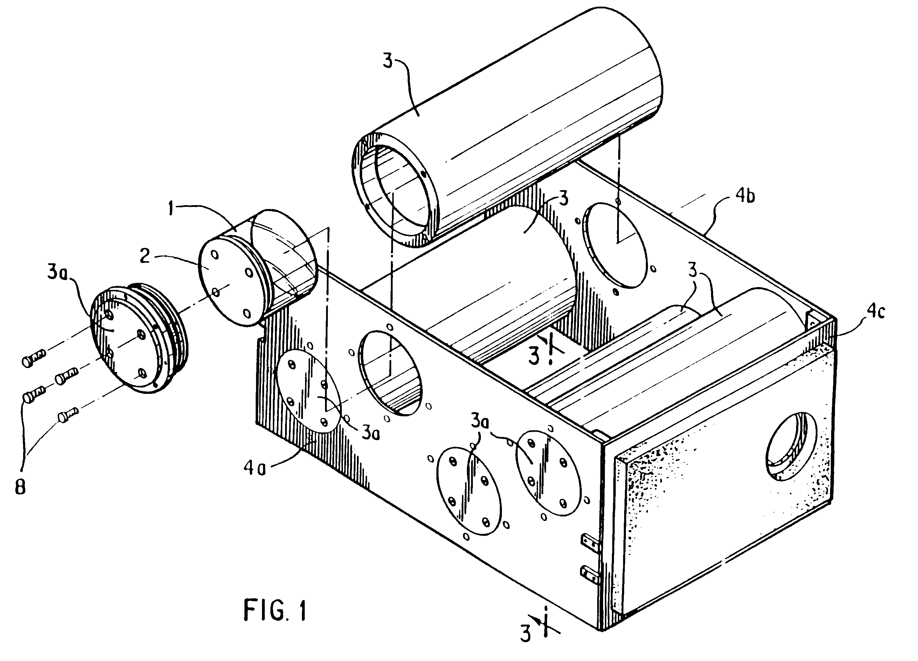

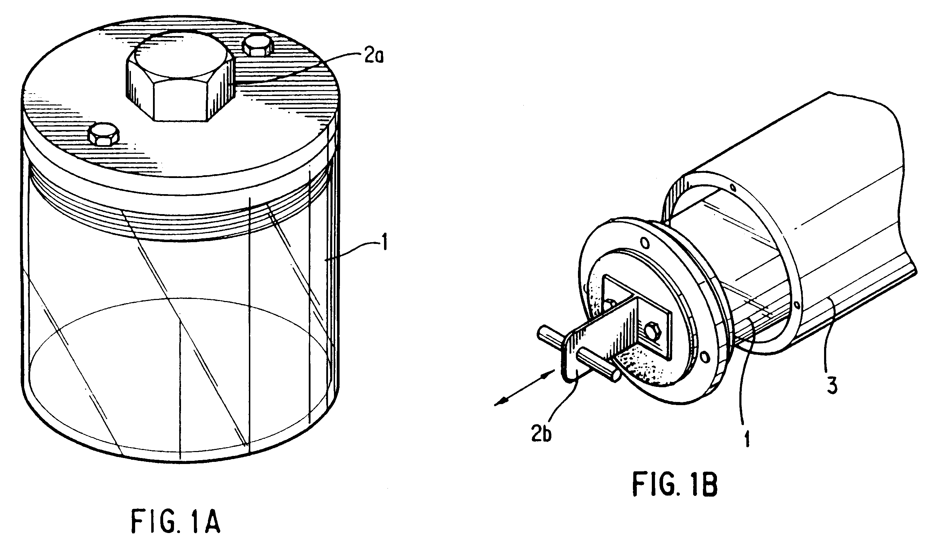

Rails 6 are bolted to the side plates 4a-b, and these rails are fitted in machined slots 12 in the RIM 5 sidewalls. The positions of the rails on the side plates can be changed to accommodate different RIMs. The whole unit is prevented from sliding out of the RIM 5 by the RIM door 5a which is bolted to the front of RIM 5. Preferably, the aluminum front plate 4c is provided with a foam layer as shown in Fig. 1 to ensure a snug fit within the RIM once the door 5a has been closed. Any foam material could be used for this layer, but PYREL® from Scott Foam is preferable as it is flame-retardant and has been approved for space flight by NASA.

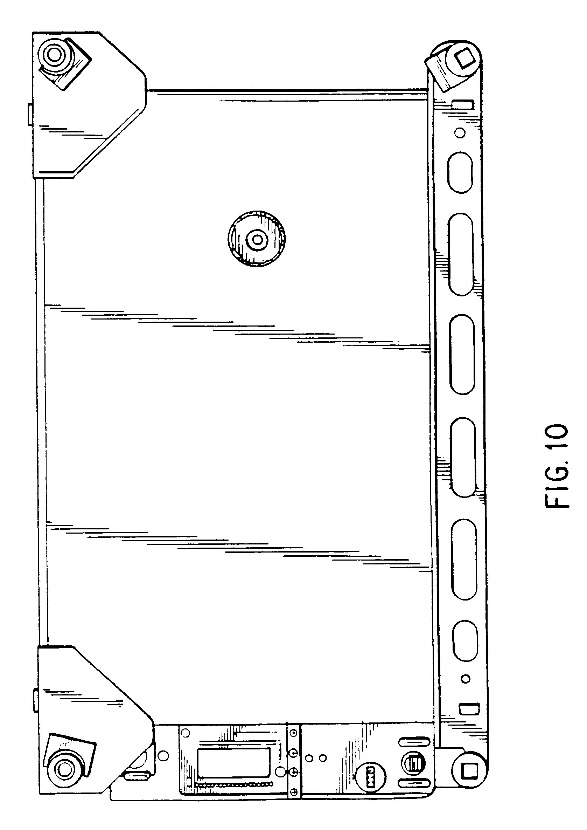

The aluminum front plate 4c is provided with a circular aperture as shown in Fig. 1. This aperture is provided to cooperate with a pressure release valve of the RIM. The pressure release valve, which is shown from the exterior of the RIM in Fig. 10, prevents implosion or explosion of the RIM when sudden changes in pressure occur.

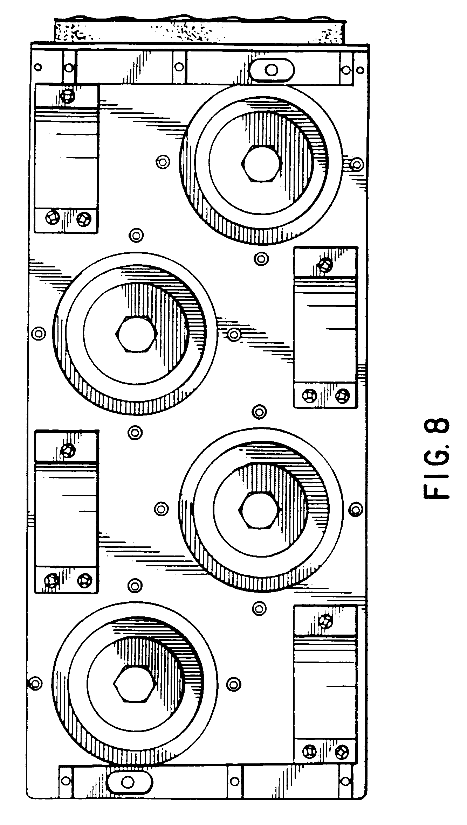

The left side plate 4a is used as the thermal plate. Since one of the primary objectives of the RIM 5 during the PCF operation is to provide identical thermal environments to the bottles, it is very important that the interface between the thermal plate and the top of the sample container be highly conductive. For this reason, the PCF is designed so each aluminum PCF cap 2 is screwed to the aluminum cylinder top 3a of the corresponding cylinder 3 and the aluminum cylinder top 3a feeds through the left side plate 4a to the thermal plate of the RIM 5, thus giving a good conductive path to the protein solution. As shown in Fig. 8, the right end plate also uses a series of leaf springs to help keep the PCF pushed up against the thermal plate of the RIM 5.

The RIM door 5a remains closed at all times when the apparatus is flown. The only required crew operation is the adjustment of a temperature control knob located on a control panel of the RIM 5. Ideally, the temperature is set at $40 °C$ while the RIM is being placed in the Orbiter and remains at that temperature during the initial phase of the flight. Then, during the first 24-48 hours of the flight, the temperature is lowered from 40°C to 22°C. After the temperature adjustment of the RIM 5 is complete, the PCF environment requires no more crew activity with the exception of occasional monitoring to ensure that the RIM 5 is holding the correct temperature.

For insulin samples, the following materials can be used: bovine insulin 24 I.U. per mg., crystalline, 0.5% zinc content (available from Sigma Chemical Co., St. Louis, MO under cat. no. 15500), protamine (salmine sulfate) from salmon, grade II (available form Sigma under cat. no. P4380) and m-cresol (3-methylphenol), approximately 99% (available from Sigma under cat. no. C5015); and sodium phosphate (dibasic heptahydrate) and sodium chloride (certified A.C.S. grade available from Fisher Scientific, Fair Lawn, NJ).

Insulin samples at 1.6 mg/ml to 0.2 mg/ml are prepared in 0.01M $Na_2HPO_4$, 0.5 M NaCl and 0.3% m-cresol, pH 6.5. The samples are incubated at 60°C for 10 minutes to facilitate the insulin dissolution and cooled at room temperature for 5 minutes afterwards. Then, a 1.0% solution of salmine sulfate is added such that the ratio of milligrams salmine sulfate to milligrams insulin is 0.125. The resulting suspension is warmed to 60°C and incubated at 60°C for 60 minutes to dissolve as much of the amorphous precipitate as possible.

The sample is filtered through a 0.22 (micron) $\mu$m nylon filter (Rainin, Woburn, MA) at 60°C and the resulting filtrate is allowed to cool slowly by placement in a programmable water bath and lowering the temperature of the bath from 60°C to 40°C in a linear fashion over 24 hours. The filtrate is then poured into four prewarmed PCF bottles 1 and the visible bubbles clinging to the inside wall are removed with a tetrafluoroethylene-coated spatula at 40°C. The insulin solution fills the bottle to such an extent that when the aluminum PCF cap 2 is screwed on there are no visible air bubbles. After the bottle is filled, the cap is screwed down to a specified torque. To facilitate this operation, a torquing tool 2a may be used. Each of the four PCF bottles 1 are filled in this manner at 40-45°C and then each is placed in the corresponding aluminum cylinder 3.

Placement and removal of the PCFs may be facilitated by using a T-handle 2b temporarily attached to the aluminum cylinder top 3a. The cylinder 3 is in turn placed in an assembly comprising the two side plates 4a-b and the front panel 4c which interfaces with the RIM 5. Again, all of these operations are done at 40-45°C.

The PCF loading procedure according to a preferred embodiment of the present invention is described in detail below:

1. The PCF bottle is filled with the insulin solution.

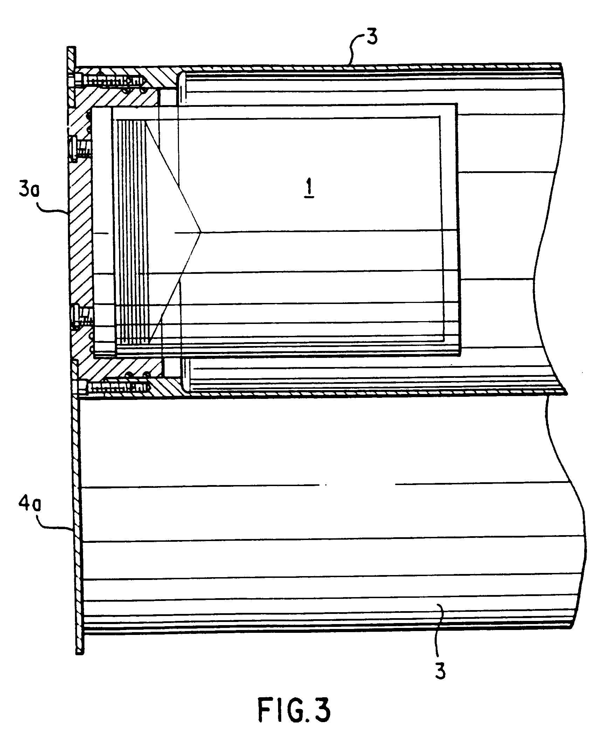

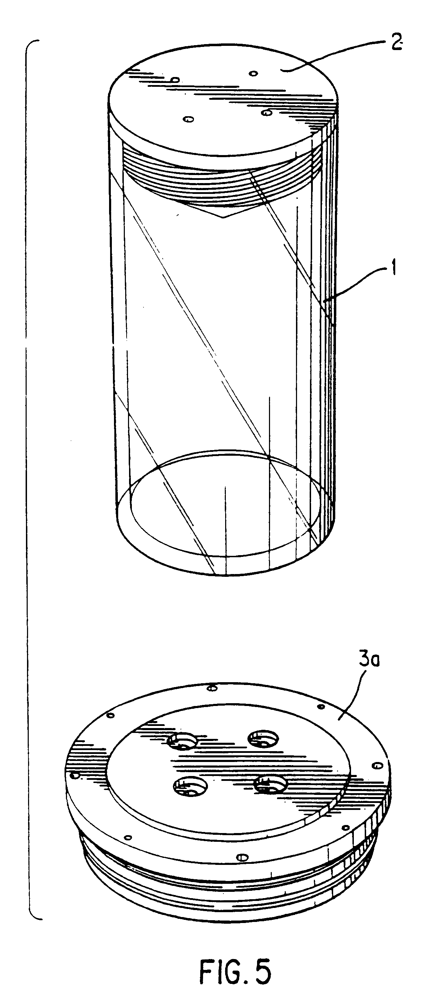

2. The aluminum cap is screwed onto the bottle. As shown in Figs. 3 and 5, the shape of the cap pushes any air bubbles out of the bottle.

3. The torquing tool is attached to the top of the cap with four screws.

4. The cap is tightened to 23.01 kg/cm (130 lbs/in.) with a torque wrench.

5. The torquing tool is detached.

6. The aluminum cylinder top is attached to the PCF aluminum cap with four screws torqued to 455g/cm (40 oz/in).

7. The assembly is placed in the aluminum cylinder.

8. The assembly is attached to the aluminum cylinder with four screws torqued to 455g/cm (40 oz/in).

9. At the end of the aluminum cylinder opposite to the PCF cap a stem-and-purge cap is attached and torqued to 455g/cm (40 oz/in).

10. Steps 1-9 are performed for the other PCFs.

11. Each aluminum cylinder is emersed in water to check for air leakage from the cylinder by watching for bubbles.

12. Once the leak test has been passed, the four aluminum cylinders are attached to the right side plate using four screws torqued to 455g/cm (40 oz/in). for each cylinder.

13. The left side plate is attached to the other end of the cylinders in the same way.

14. The left and right side plates are attached to the front panel with 6 screws torqued to 455g/cm (40 oz/in).

15. The completed assembly is placed in the RIM, which has been prewarmed to an internal temperature of 40°C.

These operations are done at 40-45°C so as to keep the insulin solution from going lower than 40°C prior to loading into the RIM, which is set at 40°C. The RIM 5 remains powered and set at 40°C until activation in microgravity. Once in orbit, the RIM 5 is cooled from 40°C to 22°C over a period of about 26 hours. Four temperature changes are made: 40-36°C at about 3 hours; 36- 32°C at about 9 hours; 32-28°C at about 19 hours; and 28-22°C at about 26 hours.

In another embodiment of the invention, the cantilevered mounting of the PCFs within the cylinders may be replaced with stiff insulation to hold the bottle in place within the cylinder. This stiff insulation can be molded or can be introduced into the cylinder as a foam which hardens around the bottle. PYREL® from Scott foam is preferable for the stiff insulation, however any material can be used having a thermal conductivity value which is less than that of the solution in the bottle. This is necessary to ensure that heat is not drawn away from the bottle.

In addition, the PCFs may be held in place using an internal brace such as a "donut" brace or a ribbed or spoked brace. Furthermore, the PCFs may be provided with lengthwise or circumferential extensions corresponding to the inner surfaces of the cylinders.

Biological macromolecules for which crystals are producible in accordance with the present invention are those naturally occurring or genetically engineered organic compounds having molecular weights of at least about 1000. Exemplary biological macromolecules include proteins, nucleic acids, proteoglycans, protein-lipid complexes, and glycoproteins. Precipitating (crystallizing) agents are those compounds that, when added to the solution of the biological macromolecule, cause the solution to become supersaturated, leading to protein-protein aggregation resulting in the formation of protein crystals.

Exemplary precipitating agents include polyethylene glycol (PEG), 2-methyl-2,4-pentanediol (MPD), ammonium sulfate, and sodium chloride. The particular agent needed for a particular biological macromolecule will be readily apparent to the skilled artisan, as demonstrated in Scopes, Protein Purification, Principles and Practice, second edition, Springer, Verlag, 1987, New York, pp. 298-299, Blundell, et al., Protein Crystallography, Academic Press, 1976, New York, pp. 59-82, and McPherson, Preparation and Analysis of Protein Crystals, John Wiley & Sons, 1982, New York, pp. 102-108.

In accordance with the method of the present invention, temperature change is used to effect crystal growth of the biological macromolecules. Both a warm-to-cold gradient and a cold-to-warm gradient can be used depending on the biological macromolecule that is to be crystallized. Temperature change is used to control crystal growth because it is noninvasive, i.e., the pH, the protein concentration, and the precipitating agent concentration do not have to be changed. Also, no seeding is involved.

Problems encountered on earth in using temperature change to grow crystals are minimized in a microgravity environment. On earth, temperature gradients can set up large convection currents, which can distort and adversely effect protein crystal growth. In a microgravity environment, temperature gradients do not cause such convection currents because of the minimal gravitational force exerted.

Temperature gradients in both directions are effective in growing protein crystals in accordance with the present invention. The temperature gradient necessary for crystallization depends upon the biological macromolecule used. Insulin and catalase, for example, crystallize by reducing the temperature of a heated solution to create a temperature gradient therein. This happens because the protein is more soluble at warmer temperatures, and as it is cooled, it crystallizes out of solution. Other proteins that are known to crystallize using temperature decrease to effect a temperature gradient can also be crystallized in accordance with the present invention.

On the other hand, effecting temperature gradients by increasing the temperature can also be used in accordance with the present invention. For example, in Jakoby, "Crystallization as a purification technique," Methods in Enzymology, Vol 11, pp. 248-252, Academic Press NY (1971), and Jakoby, Analytical Biochemistry, 26, 295-298 (1968), protein crystallization is realized as the temperature is raised from 4°C to 22°C. This depends on the differential solubility of proteins in a high salt solution as temperature increases. With this procedure, the protein of interest is dissolved at a relatively low concentration in a high concentration of ammonium sulfate at 4°C. The temperature is raised and the protein crystallizes out at the elevated temperature.

The increase in temperature increases the activity of the salt which, in turn, increases its effective concentration. This causes more interaction between the salt and the water which essentially ties up large numbers of water molecules. The effective concentration of the water thereby decreases and concomitantly the concentration of the protein increases and it crystallizes out of the solution. Examples of such proteins are alkaline phosphatase and aldehyde dehydrogenase. Other proteins known to crystallize using an increase in temperature to effect a temperature gradient can also be crystallized in accordance with the present invention.

Temperature change, and particularly temperature ramping, is the easiest way to dynamically control the system. Temperature change is noninvasive, readily engineered, and can be modeled with ground-based work. For these reasons, temperature change is an effective regulator of bulk protein crystal growth in microgravity.

In accordance with the present invention, temperature change can be effected by either manually adjusting temperature controls at given time intervals or by using computer control to establish a constant

6

temperature change over time. Preferably, such temperature change should occur at a rate of about 0.0002-0.03 °C per minute, more preferably about 0.003-0.0125 °C per minute, to effect the proper temperature gradient. Ordinarily, a change of about 18 °C in one hour gives only fair crystals, a change of about 18 °C over about 24 hours gives good crystals, and a change of about 18 °C over less than about 6 weeks is necessary to avoid degradation of the crystals. Solution conditions necessary for crystallization, such as precipitating (crystallizing) agent, other solution additives, concentration of biological macromolecule, precipitating agent, and other additives in solution, solution pH, etc, depend on the particular biological macromolecule to be crystallized and will be readily apparent to the skilled artisan.

In accordance with the method and apparatus of the present invention, formation of varying amounts of crystals are possible. Preferably, crystallization conditions are adjusted in accordance with the present invention to produce commercial scale quantities, i.e., at least 0.5 mg, 1.0 mg, 500 mg, or 1000 mg of crystals, including gram quantities and kilogram quantities. Preferably, solution volumes of at least 5 ml, more preferably at least 10 ml, and concentration levels of biological macromolecule of at least 0.2 mg/ml or at least 5 mg/ml are contemplated. Particular conditions necessary to effect these amounts depend on the biological macromolecule to be crystallized, and will be readily apparent to skilled artisan.

Microgravity conditions allow the crystals produced in accordance with the present invention to exhibit more order than their earth-grown counterparts. That is, the microgravity-grown crystal produces a higher Bragg angle at its diffraction limit (i.e., the limit of statistically usable data) as compared to earth-grown crystals and/or the microgravity-grown crystals display less thermal vibration of atomic and molecular lattice misorientation as reflected by the positive $\Delta B$ slope displayed on a relative Wilson plot when compared to earth-grown crystals of the same biological macromolecule.

The Bragg angle and Wilson plot are well known bases for determining the relative order in crystals. DeLucas, et al., Science, 246, 651-654 (1989). In X-ray diffraction analysis of crystals, the Bragg angle $\theta$ is the angle at which X-ray diffraction data is generated, which is used to calculate $1/d^2$ x $10^{-10}$ $m^2(Å^2)$, where d is the calculated distance between planes in the crystal, according to the equation $1/d^2 = 4 \sin^2\theta\lambda^2$, where $\lambda$ is typically the wavelength equal to 1.5418 x $10^{-10}$ $m^2$ (Å).

For example, at their diffraction limit, $1/d^2$ for crystals for gamma-interferon is 0.11 x $(10^{-10}m)^{-2}$ and for earth-grown crystals is 0.09 x $(10^{-10}m)^{-2}$. The $1/d^2$ values correspond to a limit of resolution for the microgravity-grown crystals of 3.0 x$10^{-10}$ m (Å), and for the earth grown crystals of 3.3 x$10^{-10}$ m (Å). This means that one can decipher molecular detail as small as 3.0 x$10^{-10}$ m (Å) in the microgravity-grown crystals, while the smallest molecular detail decipherable in the earth-grown crystals is 3.5 x$10^{-10}$ m (Å). For crystals of porcine elastase, $1/d^2$ is 0.41 x $(10^{-10}m)^{-2}$ and the resolution limit 1.56 x10-10 m (Å) for microgravity-grown crystals and $1/d^2$ is 0.32 x $(10^{-10}m)^{-2}$ and the resolution limit 1.76 x$10^{-10}$ m (Å) for earth-grown crystals. For crystals of isocitrate lyase, $1/d^2$ is 0.19 x $(10^{-10}m)^{-2}$ and the resolution limit 2.3 x$10^{-10}$ m (Å) for microgravity-grown crystals,and $1/d^2$ is 0.13 x $(10^{-10}m)^{-2}$ and the resolution limit 2.8 x$10^{-10}$ m (Å) for earth-grown crystals.

Values are taken from the relative Wilson plot of particular crystals to calculate the crystal disorder produced either by thermal vibration or molecular misorientations. The value **B** reflects thermal vibration, atomic and molecular lattice misorientations and is calculated from the slope of a relative Wilson plot according to the formula Slope = $\Delta B/2$, where $\Delta B$ is the difference between the B value of the earth-grown crystals and microgravity-grown crystals, with a positive $\Delta B$ indicating lower thermal vibration for the microgravity-grown crystals. For gamma interferon, $\Delta B$ is 5 units, where the units are $10^{-10}$ $m^2$ $(Å^2)$ between the resolution range of 6.0 and 3.65 x$10^{-10}$ m (Å). The absolute value of the B factor for most crystals is between 10 and 20 units. In the resolution range of 3.65-2.9 x$10^{-10}$ m (Å), $\Delta B$ is 18.5 units. For porcine elastase, $\Delta B$ is 5.2 units in the resolution range of 2-1.7 x $10^{-10}$ m (Å). For isocitrate lyase, $\Delta B$ is 6.2 units in the resolution range of 5-2.5 x$10^{-10}$ m (Å).

Example 1

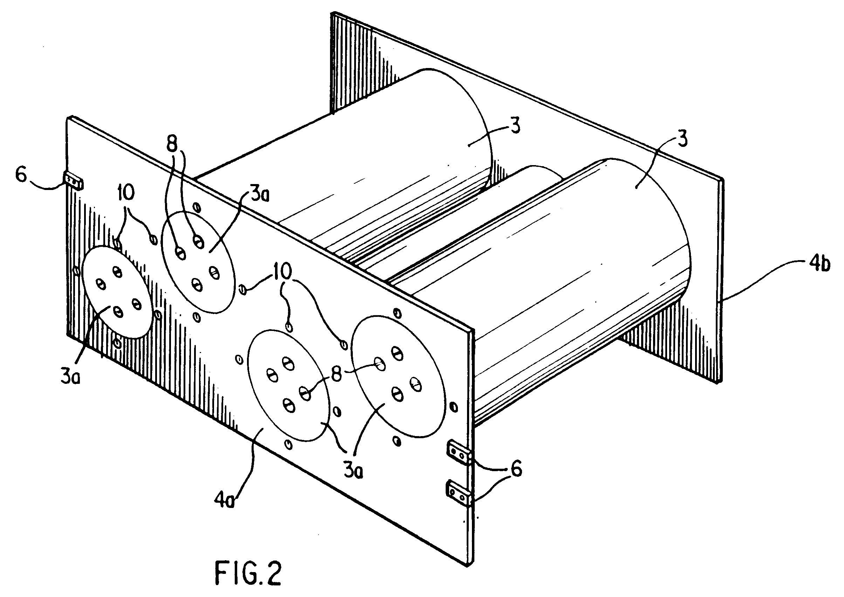

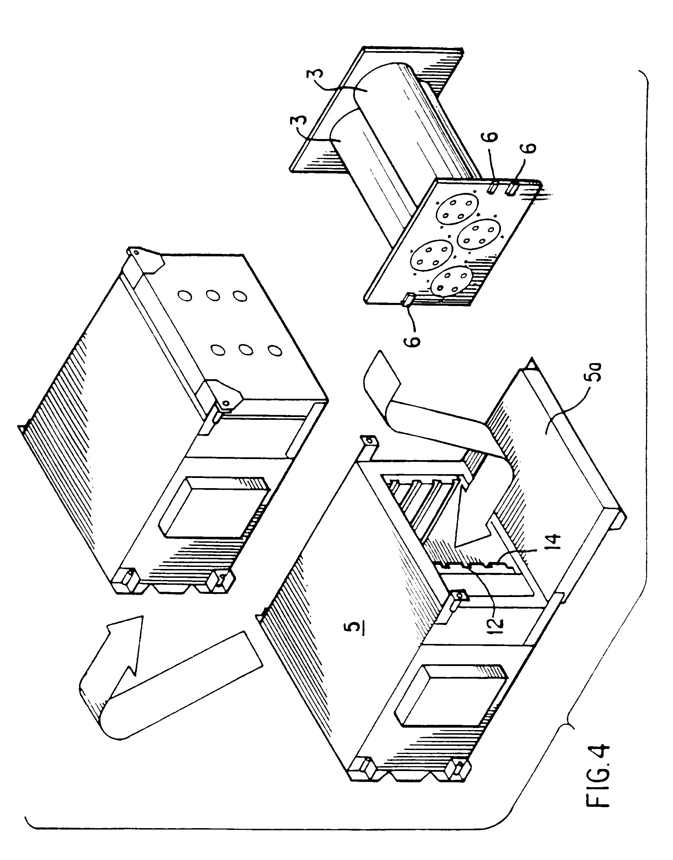

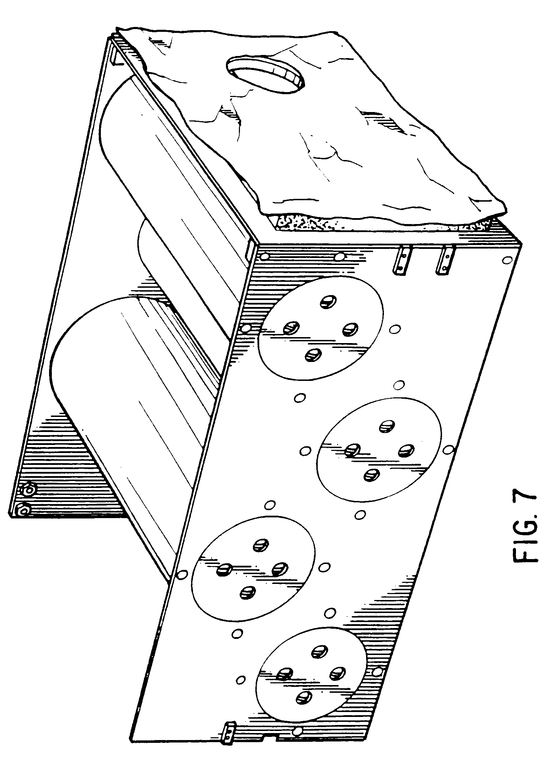

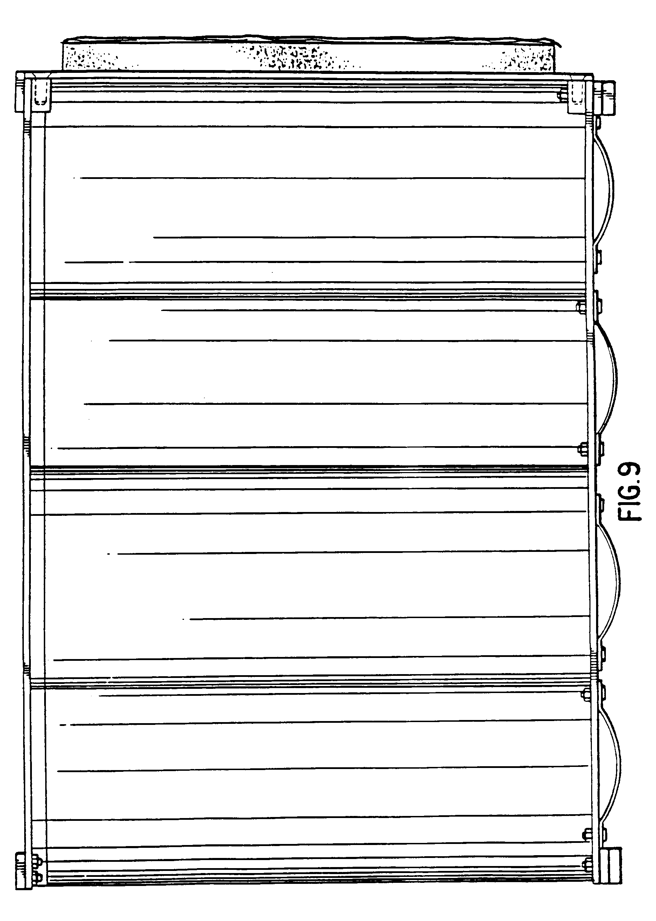

An experiment was conducted using the apparatus shown in Figures 1-10. Insulin having a concentration of 0.4 mg/ml, in a phosphate buffer, was poured into the PCF bottles. At launch plus three hours, the temperature was lowered from 40 °C to 36 °C. At launch plus nine hours, the temperature was lowered from 36 °C to 32 °C. At launch plus 19 hours, the temperature was lowered from 32 °C to 28 °C. At launch plus 26 hours, the temperature was lowered from 28 °C to 22 °C.

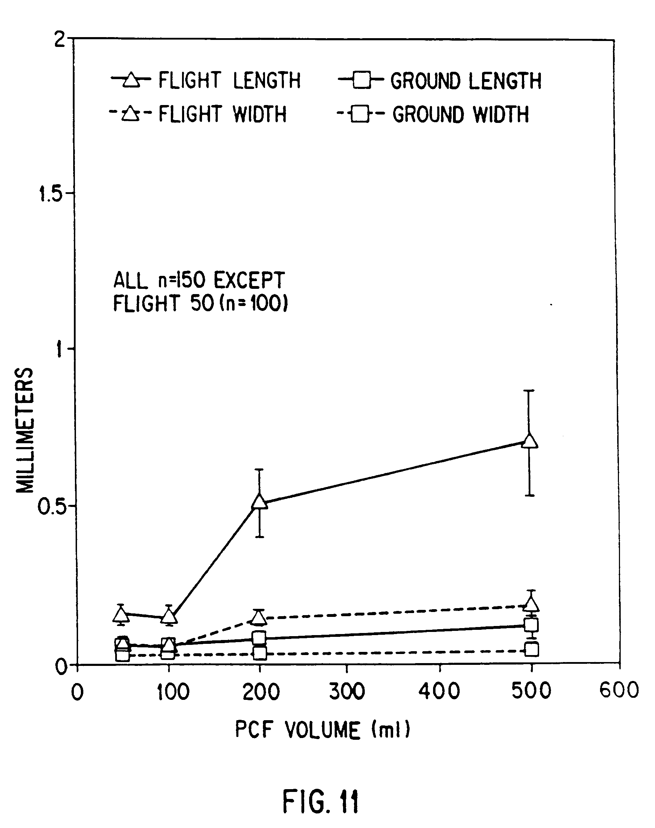

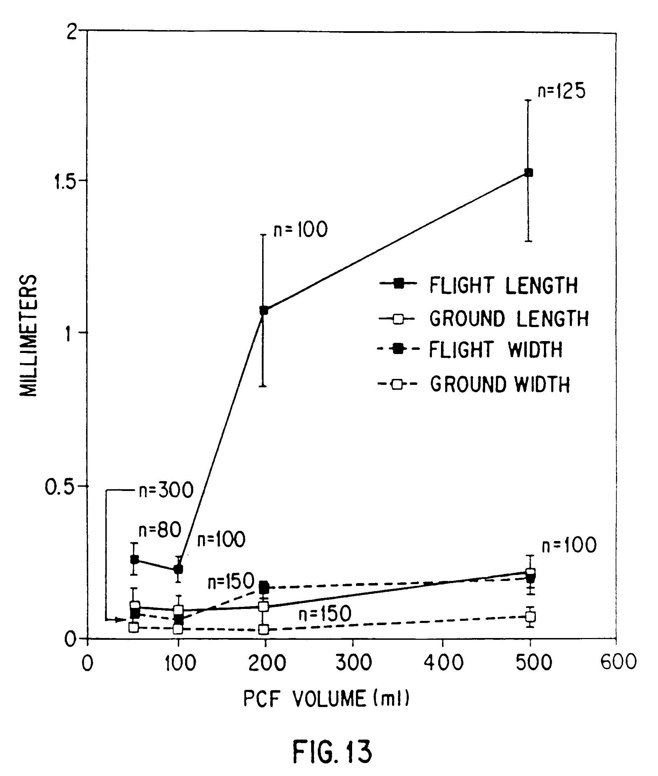

The length and width of "rosette" and single crystals grown in flight were measured and compared to the length and width of reference "rosette" and single crystals grown on the ground under otherwise similar conditions. As shown in Figures 11 and 13, the free-floating crystals grown in flight were longer and wider than those grown on the ground. In fact, the ratio of free-floating flight "rosette" crystal size to free-floating ground "rosette" crystal size was found to be:

| PCF | Length | Width |
|-----|--------|-------|
| 500 | 5.94 | 4.13 |
| 200 | 4.71 | 3.43 |
| 100 | 2.40 | 1.74 |
| 50 | 2.76 | 2.34 |

The ratio of free-floating flight single crystal size to free- floating ground single crystal size was found to be:

| PCF | Length | Width |
|-----|--------|-------|
| 500 | 10.26 | 3.96 |
| 200 | 8.66 | 3.66 |
| 100 | 2.42 | 1.84 |
| 50 | 2.42 | 2.24 |

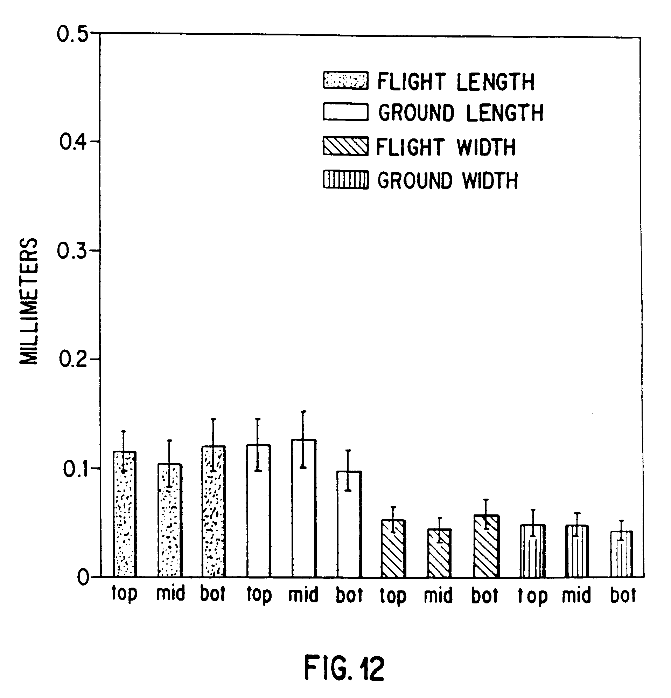

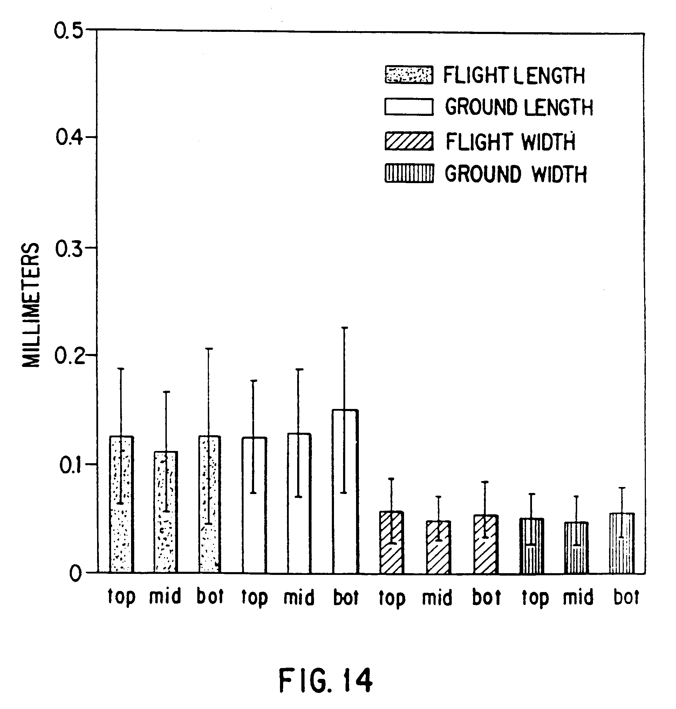

In addition to the crystals which were found free-floating in the PCF bottles, some crystals were found to be attached to the side of the bottles. As can be seen from Figures 12 and 14, there did not appear to be a significant difference between the size of the crystals and their location along the longitudinal axis of the PCF bottle. X-ray crystallography indicated that the space grown crystals diffracted to higher resolution than the earth grown controls.

Example 2

Another experiment was conducted using the apparatus shown in Figures 1-10. Insulin having a concentration of 0.4 mg/ml, in a phosphate buffer, was poured into the PCF bottles. At launch plus four hours, the temperature was lowered from 40°C to 22°C.

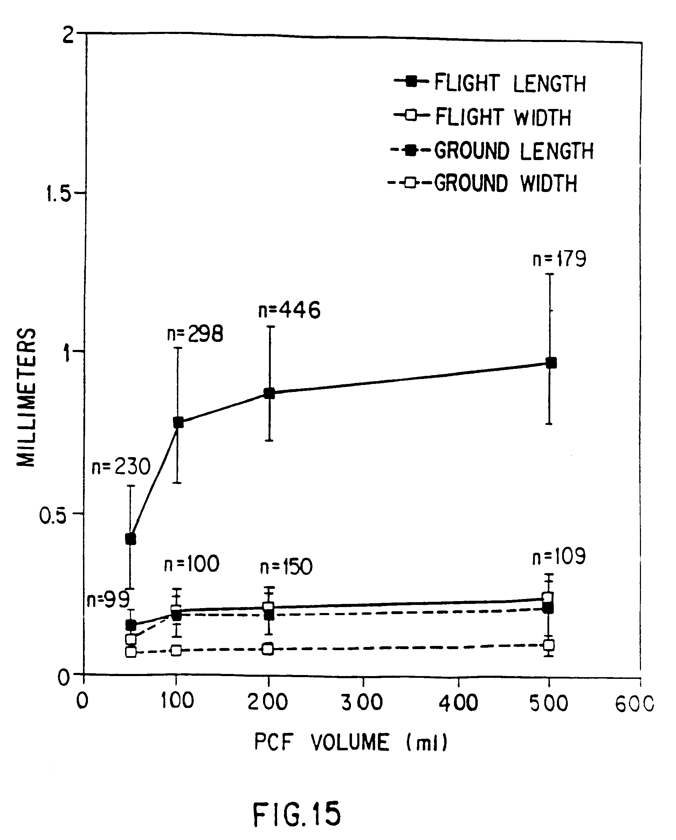

As shown in Figure 15, the free-floating crystals grown in flight were longer and wider than those grown on the ground. In fact, the ratio of free-floating flight single crystal size to free-floating ground single crystal size was found to be:

| PCF | Length | Width |
|-----|--------|-------|
| 500 | 4.58 | 2.55 |
| 200 | 4.65 | 2.64 |
| 100 | 4.12 | 2.68 |
| 50 | 2.76 | 1.65 |

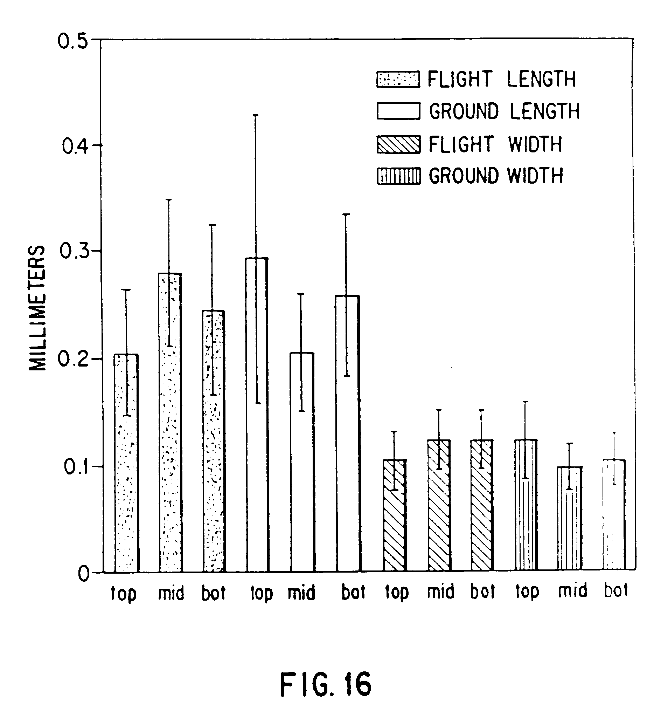

As can be seen from Figure 16, again there did not appear to be a significant difference between the size of the crystals attached to the side of the PCF bottle and their location along the longitudinal axis of the PCF bottle. X-ray crystallography indicated that the space grown crystals diffracted to higher resolution than their earth grown controls.

While there are shown and described present embodiments of the invention, it is to be distinctly understood that the invention is not limited thereto, but may be otherwise variously embodied and practiced within the scope of the following claims.

**Claims**

1. An apparatus capable of producing macromolecular crystals in microgravity comprising:
   (a) at least one container having opposing ends and a thermally conductive lid at one of said opposing ends;
   (b) at least one bottle disposed inside the container and having a thermally conductive cap such that the cap contacts the lid so as to allow heat transfer therebetween; and

(c) means for maintaining the lid in contact with a variable heat source such that a temperature gradient is obtainable inside said bottle.

2. The apparatus of claim 1, wherein said at least one bottle is made of polysulfone.

3. The apparatus of claim 1 or 2, wherein a bottom portion of said thermally conductive cap is substantially V-shaped.

4. The apparatus of any one of claims 1 to 3 having a plurality of bottles, each of which is disposed in a separate container.

5. The apparatus of claim 4, wherein said plurality of bottles are of different volumes so that a different temperature gradient is obtainable in each bottle.

6. The apparatus of claim 4 or 5, wherein said plurality of bottles are identical in circumference and different in volume.

7. The apparatus of any one of claims 1 to 6 wherein the cap and lid are at least partly made of metal such that temperature changes are transmitted to the inside of the bottle from the variable heat source through the metal.

8. A method for producing commercial scale quantities of macromolecular crystals of at least 0.5 mg comprising:
   (a) providing at least one bottle having a thermally conductive cap;
   (b) loading a pre-mixed aqueous solution of a liquid biological macromolecule and a precipitating agent into the bottle and fixing said cap onto said bottle after loading the solution into said bottle;
   (c) placing said thermally conductive cap in contact with a variable heat source such that the cap contacts the variable heat source so as to allow heat transfer therebetween; and
   (d) changing the temperature of said variable heat source while said bottle is in the microgravity environment so as to establish a temperature gradient in the bottle sufficient to crystallize the biological macromolecule wherein the microgravity grown crystals thereby produced are characterized by at least one of the group consisting of higher Bragg angles for the diffraction limit, less thermal vibration, and less atomic and molecular lattice misorientation as reflected by the positive $\Delta B$ slope displayed on a relative Wilson plot when compared to earth-grown crystals of the same biological macromolecule.

9. The method of claim 8 wherein the volume of the aqueous solution is at least 5 ml and the concentration of the biological macromolecule is at least 0.2 mg/ml.

10. The method of claim 8 or 9 wherein the temperature is decreased to effect the temperature gradient.

11. The method of claim 8 or 9 wherein the temperature is increased to effect the temperature gradient.

12. The method of any one of claims 8 to 11 comprising the steps of:
   (a) loading the solution of the liquid biological macromolecule and crystallizing agent into a plurality of bottles of different volume each having a thermally conductive cap;
   (b) maintaining the thermally conductive caps in contact with a variable heat source; and
   (c) changing the temperature of said variable heat source while the bottles are in the microgravity environment so as to establish a different temperature gradient in each bottle.

**Patentansprüche**

1. Vorrichtung, die zur Herstellung von Makromolekül-Kristallen in der Fast-Schwerelosigkeit geeignet ist, umfassend:
   (a) wenigstens einen Behälter mit gegenüberliegenden Enden und einen wärmeleitfähigen Deckel an einem der gegenüberliegenden Enden;
   (b) wenigstens eine Flasche, die im Behälter angeordnet ist und die einen wärmeleitfähigen Verschluß hat, so daß der Verschluß den Deckel berührt, so daß ein Wärmeübergang dazwischen ermöglicht wird; und
(c) Mittel, wodurch der Deckel in Berührung mit einer variablen Wärmequelle gehalten wird, so daß innerhalb der Flasche ein Temperaturgradient erhältlich ist.

2. Vorrichtung nach Anspruch 1, wobei wenigstens eine Flasche aus Polysulfon hergestellt ist.

3. Vorrichtung nach Anspruch 1 oder 2, wobei ein Bodenteil des wärmeleitfähigen Verschlusses im wesentlichen eine V-Form aufweist.

4. Vorrichtung nach einem der Ansprüche 1 bis 3 mit einer Mehrzahl von Flaschen, von denen jede in einem separaten Behälter angeordnet ist.

5. Vorrichtung nach Anspruch 4, wobei die Mehrzahl von Flaschen unterschiedliche Volumina aufweisen, so daß in jeder Flasche ein verschiedener Temperatur-Gradient erhältlich ist.

6. Vorrichtung nach Anspruch 4 oder 5, wobei die Mehrzahl von Flaschen identisch im Umfang und unterschiedlich im Volumen sind.

7. Vorrichtung nach einem der Ansprüche 1 bis 6, wobei der Verschluß und der Deckel wenigstens teilweise aus Metall hergestellt sind, so daß von der variablen Wärmequelle durch das Metall Temperaturänderungen in das Innere der Flasche übertragen werden.

8. Verfahren zur Herstellung von Makromolekül-Kristallen von wenigstens 0,5 mg in kommerziellen Größenordnungen, umfassend:
(a) Bereitstellen wenigstens einer Flasche mit einem wärmeleitfähigen Verschluß;
(b) Füllen einer vorgemischten wässrigen Lösung eines flüssigen biologischen Makromoleküls und eines Fällungsmittels in die Flasche und das Befestigen des Verschlusses auf der Flasche, nachdem die Lösung in die Flasche gefüllt ist;
(c) In-Berührung-Bringen des wärmeleitfähigen Deckels mit einer variablen Wärmequelle, so daß der Deckel in Berührung mit der variablen Wärmequelle ist, so daß der Wärmeübergang dazwischen ermöglicht ist; und
(d) Änderung der Temperatur der variablen Wärmequelle, während sich die Flasche in der Umgebung der Fast-Schwerelosigkeit befindet, so daß ein Temperaturgradient in der Flasche etabliert wird, der ausreichend ist, um das biologische Makromolekül darin zu kristallisieren, wobei die in der Fast-Schwerelosigkeit gezüchteten Kristalle, die so hergestellt wurden, durch wenigstens eine der aus den höheren Bragg-Winkeln der Brechungsgrenze bestehende Gruppe ohne thermische Schwingung und ohne atomare und molekulare Gitter-Fehlordnung, wie dies durch die positive ΔB-Neigung, angezeigt auf einem relativen Wilson-Diagramm, wiedergespiegelt wird, wenn sie mit auf der Erde gezüchteten Kristallen desselben biologischen Makromoleküls verglichen werden, charakterisiert werden.

9. Verfahren nach Anspruch 8, wobei das Volumen der wässrigen Lösung wenigstens 5 ml beträgt und die Konzentration des biologischen Makromoleküls wenigstens 0,2 mg/ml beträgt.

10. Verfahren nach Anspruch 8 oder 9, wobei die Temperatur erniedrigt wird, um den Temperaturgradienten zu bewirken.

11. Verfahren nach Anspruch 8 oder 9, wobei die Temperatur erhöht wird, um den Temperaturgradienten zu bewirken.

12. Verfahren nach einem der Ansprüche 8 bis 11, umfassend die Schritte des:
(a) Füllens der Lösung des flüssigen biologischen Makromoleküls und des Kristallisationsmittels in eine Mehrzahl von Flaschen mit unterschiedlichem Volumen, von denen jede einen wärmeleitfähigen Verschluß hat;
(b) In-Berührung-Halten der wärmeleitfähigen Verschlüsse mit einer variablen Wärmequelle; und
(c) Ändern der Temperatur der variablen Wärmequelle, während die Flaschen sich in der Umgebung der Fast-Schwerelosigkeit befinden, wodurch in jeder Flasche ein unterschiedlicher Temperaturgradient etabliert wird.

EP 0 573 599 B1

**Revendications**

1. Appareil capable de produire des cristaux macromoléculaires en microgravité, comprenant :

   (a) au moins un conteneur ayant des extrémités opposées et un couvercle thermiquement conducteur à l'une desdites extrémités opposées;

   (b) au moins une bouteille disposée à l'intérieur du conteneur et ayant un capuchon thermiquement conducteur de manière que le capuchon contacte le couvercle afin de permettre le transfert de chaleur entre eux; et

   (c) un moyen pour maintenir le couvercle en contact avec une source de chaleur variable telle que l'on puisse obtenir un gradient de température à l'intérieur de ladite bouteille.

2. Appareil de la revendication 1, où ladite au moins une bouteille est faite en polysulfone.

3. Apppareil de la revendication 1 ou 2, où le fond dudit capuchon thermiquement conducteur est sensiblement en forme de V.

4. Appareil selon l'une quelconque des revendications 1 à 3, ayant un certain nombre de bouteilles, dont chacune est disposée dans un conteneur séparé.

5. Appareil de la revendication 4, où les bouteilles ont des volumes différents de façon à pouvoir obtenir dans chaque bouteille un gradient différent de température.

6. Appareil de la revendication 4 ou 5, où lesdites bouteilles sont identiques en circonférence et différentes en volume.

7. Appareil selon l'une quelconque des revendications 1 à 6, où le capuchon et le couvercle sont au moins partiellement faits en métal de manière que des changements de température soient transmis à l'intérieur de la bouteille à partir de la source de chaleur variable à travers le métal.

8. Méthode de production de quantités à l'échelle commerciale de cristaux macromoléculaires d'au moins 0,5 mg, consistant à :

   (a) prévoir au moins une bouteille ayant un capuchon thermiquement conducteur;

   (b) introduire une solution aqueuse pré-mélangée d'une macromolécule biologique liquide et d'un agent précipitant dans la bouteille et fixer ledit capuchon sur ladite bouteille après avoir introduit la solution dans ladite bouteille;

   (c) placer ledit capuchon thermiquement conducteur en contact avec une source de chaleur variable de manière que le capuchon contacte la source de chaleur variable, afin de permettre le transfert de chaleur entre eux; et

   (d) changer la température de ladite source de chaleur variable, tandis que ladite bouteille est dans l'environnement de microgravité, afin d'établir un gradient de température dans la bouteille qui est suffisant pour cristalliser la macromolécule biologique, où les cristaux développés en microgravité ainsi produits sont caractérisés par au moins l'un du groupe consistant en angles plus grands de Bragg pour la limite de diffraction, moins de vibration thermique et moins de défaut d'orientation du réseau atomique et moléculaire, comme cela est réfléchi par la pente positive de $\Delta B$ présentée sur un diagramme relatif de Wilson, en comparaison avec les cristaux développés sur terre de la même macromolécule biologique.

9. Méthode de la revendication 8, où le volume de la solution aqueuse est d'au moins 5 ml et la concentration de la macromolécule biologique est d'au moins 0,2 mg/ml.

10. Méthode de la revendication 8 ou 9, où la température est diminuée pour obtenir le gradient de température.

11. Méthode de la revendication 8 ou 9, où la température est augmentée pour obtenir le gradient de température.

12. Méthode selon l'une quelconque des revendications 8 à 11, comprenant les étapes de :

11

(a) introduire la solution de la macromolécule biologique liquide et de l'agent cristallisant dans un certain nombre de bouteilles de volumes différents, chacune ayant un capuchon thermiquement conducteur;

(b) maintenir les capuchons thermiquement conducteurs en contact avec une source de chaleur variable; et

(c) changer la température de ladite source de chaleur variable, alors que les bouteilles sont dans l'environnement de microgravité, afin d'établir un gradient différent de température dans chaque bouteille.

FIG. 1

FIG. 1A

FIG. 1B

EP 0 573 599 B1

FIG.2

**FIG.3**

FIG. 4

FIG. 5

FIG. 6

FIG. 7

FIG. 8

FIG.9

FIG. 10

FIG. 11

FIG. 12

FIG. 13

FIG. 14

FIG.15

FIG. 16